# EUROPEAN PATENT SPECIFICATION

(11) **EP 2 570 367 B1**
(45) Date of publication and mention of the grant of the patent: **19.10.2016**
(21) Application number: 11780684.4
(22) Date of filing: 12.05.2011
(51) Int. Cl.: B65D 85/16, A61F 13/15, A61F 13/472, B65D 5/42, B65D 77/04, B65D 77/30, B65D 77/40, A61F 13/551, B65D 5/72, B65D 75/58, B65D 77/06

(54) **PACKAGE FOR INDIVIDUALLY PACKAGED GOOD CONSISTING OF ABSORBENT ARTICLE**
PAKET AUS EINEM SAUGFÄHIGEN PRODUKT FÜR EINZELN VERPACKTE WAREN
EMBALLAGE POUR UN PRODUIT EMBALLÉ INDIVIDUELLEMENT CONSTITUÉ D'UN ARTICLE ABSORBANT

(30) Priority: 13.05.2010 JP 2010111363
(43) Date of publication of application: 20.03.2013
(73) Proprietor: Unicharm Corporation, Ehime 799-0111 (JP)
(72) Inventor: NOGUCHI, Junichi, Kanonji-shi Kagawa 769-1602 (JP); NODA, Yuki, Kanonji-shi Kagawa 769-1602 (JP)
(74) Representative: Dolleymores
(86) International application number: PCT/JP2011/060955
(87) International publication number: WO 2011/142427

(56) References cited:
- WO-A1-2005/055901
- JP-A- 7 187 197
- JP-A- 11 105 957
- JP-A- 2003 104 472
- JP-A- 2006 526 550
- JP-A- 2007 153 422
- JP-A- 2007 153 422
- JP-A- 2009 126 555
- JP-A- 2009 126 555
- US-A1- 2002 148 749
- US-A1- 2004 238 393

## Description

### TECHNICAL FIELD

The present invention relates to a package for individually packaged good of absorbent article such as sanitary napkin.

### BACKGROUND ART

An absorbent article such as sanitary napkin has been marketed in a form of individually packaged good obtained by packaging the absorbent article in a state of being folded into a predetermined shape with a sheet member. Such an individually packaged good is marketed in a state of a package in which a plurality of individually packaged goods is housed in a flexible bag body.

However, with the package, the bag body may be damaged when an external force is applied thereto during storage and transportation thereof. In addition, the individually packaged goods housed in the bag body may be deformed due to the external force applied to the bag body.

Given this, the present applicant has previously proposed a package in which a plurality of individually packaged goods of absorbent article is housed in a box body with an openable upper face configured such that a width thereof increases gradually from bottom to top (see Patent Document 1).

With the package, since the plurality of individually packaged goods is housed in the box body, deformation of the individually packaged goods due to an external force applied to the package can be prevented. In addition, since the box body is configured such that the width thereof increases from bottom to top, the absorbent article can be easily taken out from the upper face being opened.

Patent Document 1: Japanese Unexamined Patent Application Publication No. 2004-307044

A further prior art arrangement is known from JP 2009 126555.

### DISCLOSURE OF THE INVENTION

### Problems to be Solved by the Invention

In the package proposed in Patent Document 1, if most of the individually packaged goods are taken out and there is only a few individually packaged goods left in the box body, a degree of freedom of movement of the individually packaged goods inside the box body increases. As a result, if there are only a few individually packaged goods left in the box body, the individually packaged goods may move inside the box body and make it difficult to pick up the individually packaged good.

Given this, the present invention is aimed at providing a package for individually packaged good of absorbent article that allows an easy taking out of individually packaged goods housed in a box body.

### Means for Solving the Problems

According to the present invention in a first aspect, there is provided a package for individually packaged good of an absorbent article as recited by Claim 1.

It is preferable that the absorbent article has an elongated absorbent main body; the individually packaged good is configured to be packaged with the sheet member in a state in which the absorbent article is folded in a longitudinal direction at a fold line along a width direction of the absorbent main body; and the pair of extended portions is provided at the pair of side portions extending in the longitudinal direction of the absorbent article and configured such that portions of the sheet member extending from the pair of side portions are joined.

It is preferable that the bag body is housed in the box body in a state in which the plurality of individually packaged goods is stacked in a height direction of the box body.

It is preferable that the bag body has a first perforated line formed on an upper face thereof in a state of being housed in the box body.

It is preferable that the bag body further has a second perforated line formed on a side face thereof, the second perforated line being connected to the first perforated line.

It is preferable that the first perforated line extends in a direction substantially orthogonal to a direction across the pair of extended portions.

It is preferable that the first perforated line extends in a direction across the pair of extended portions.

It is preferable that the bag body further has a third perforated line that is formed on a side face of the bag body, the third perforated line extending in a direction substantially orthogonal to a height direction of the bag body.

It is preferable that the bag body is formed of a white sheet-like member.

It is preferable that the box body includes a push-up portion formed on a bottom face thereof, the push-up portion allowing push-up of the bag body.

### Effects of the Invention

In the package for individually packaged good of absorbent article according to the present invention, the individually packaged goods housed in a box body can be easily taken out.

### BRIEF DESCRIPTION OF THE DRAWINGS

FIG. 1 is a perspective view illustrating a first embodiment of the package for individually packaged good of absorbent article of the present invention;
FIG. 2 is a perspective view illustrating a bag body according to the first embodiment;
FIG. 3 is a plan view illustrating a folded state of the individually packaged good according to the first embodiment;
FIG. 4 is a developed plan view of the individually packaged good shown in FIG. 3;
FIG. 5 is a diagram showing correspondence between the plan view of the bag body shown in FIG. 2 and a plan view of the box body;
FIG. 6 is a sectional view taken along the line X-X of FIG. 5;
FIG. 7 is a plan view of FIG. 1;
FIG. 8 is a sectional view taken along the line Y-Y of FIG. 7;
FIG. 9 is a perspective view illustrating a bag body according to a second embodiment;
FIG. 10 is a perspective view illustrating a bag body according to a third embodiment;
FIG. 11 is a perspective view illustrating a bag body according to a fourth embodiment;
FIG. 12 is a plan view of the bag body shown in FIG. 11;
FIG. 13 is a bottom view of the box body according to a fifth embodiment; and
FIG. 14 is a diagram illustrating the package for individually packaged good of absorbent article according to the fifth embodiment, in use.

### DETAILED DESCRIPTION OF THE INVENTION

Preferred embodiments of the package for individually packaged good of absorbent article (hereinafter also referred to simply as "package") according to the present invention are explained hereinafter with reference to the drawings.

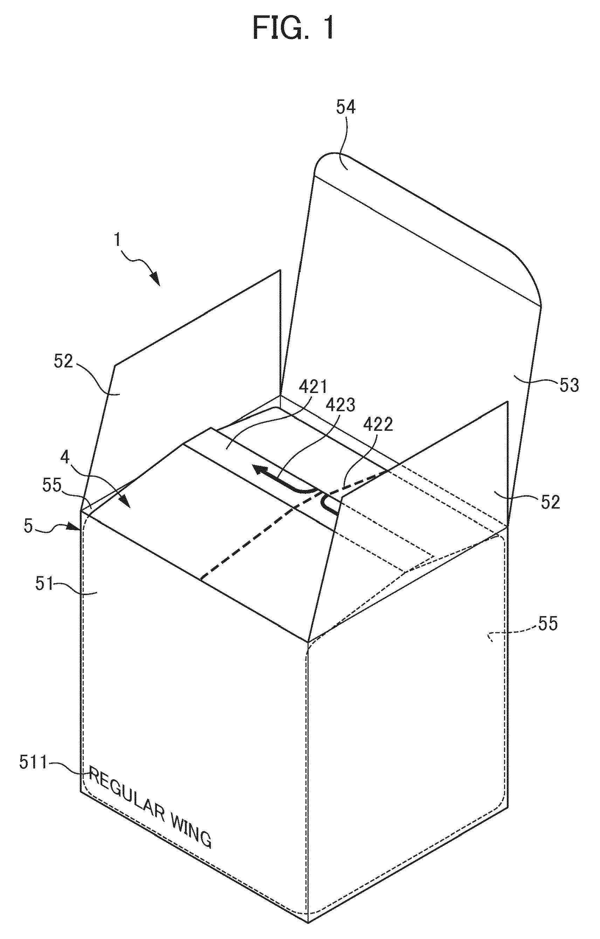

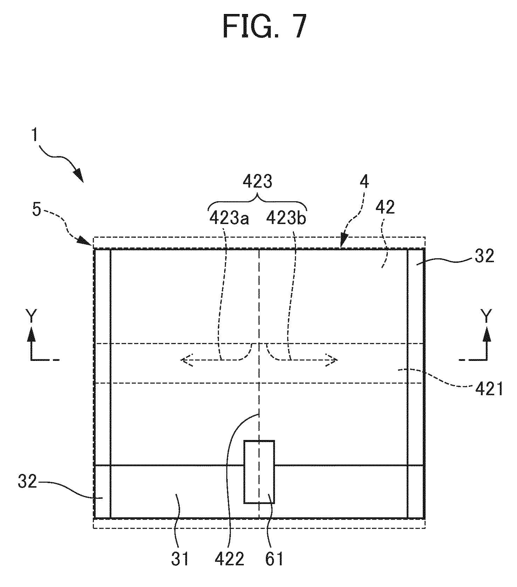

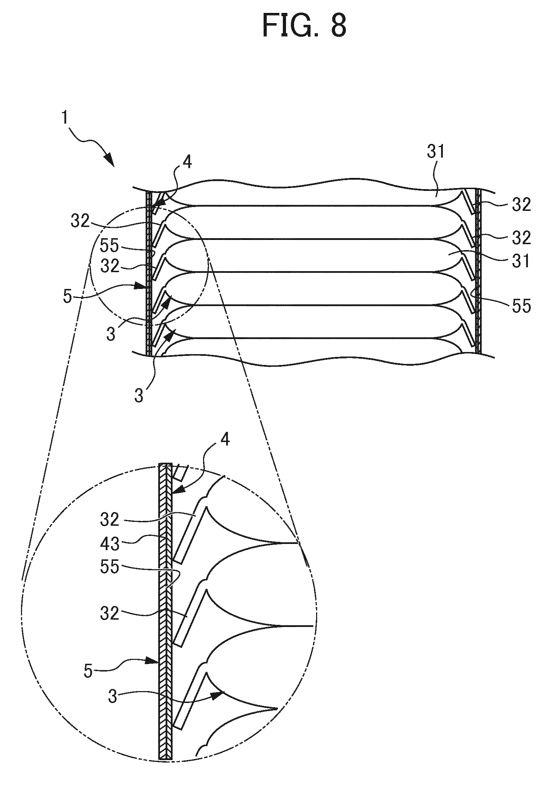

First, the package of the first embodiment is explained hereinafter with reference to FIGS. 1 to 8.

As shown in FIGS. 1 to 4, a package 1 of the first embodiment is a package that houses an individual packaged good 3 of a sanitary napkin 2 as the absorbent article. The package 1 is configured to include: the individual packaged good 3 of sanitary napkin 2; the bag body 4 that houses a plurality of individual packaged goods 3; and a box body 5 that houses the bag body 4.

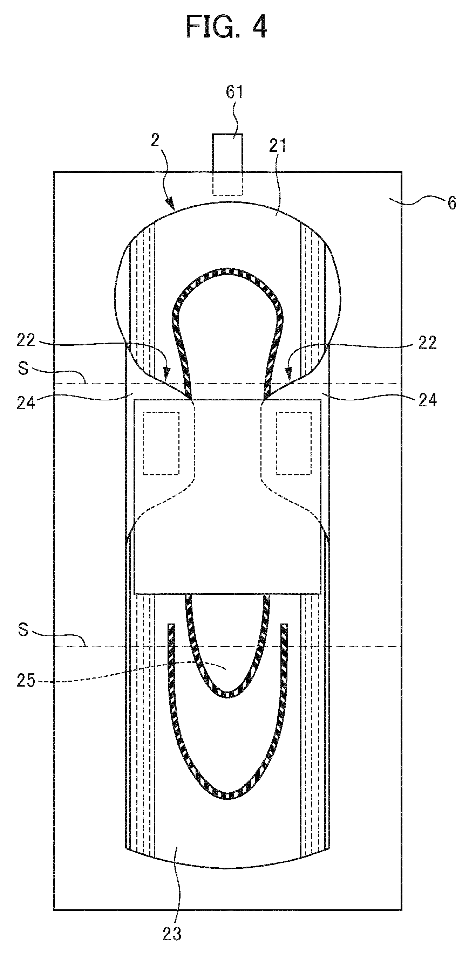

As shown in FIG. 4, the individual packaged good 3 is configured to include: the sanitary napkin 2; a sheet member 6 that packages the sanitary napkin 2; and a fixing tape 61 attached to the sheet member 6.

As shown in FIG. 4, the sanitary napkin 2 is provided with an elongated absorbent main body 21 and a pair of wing portions 22 that extends outward in a width direction, from central parts in a longitudinal direction on both sides of the absorbent main body 21.

The absorbent main body 21 includes a liquid-permeable top sheet 23, a liquid-impermeable back surface sheet 24, and a liquid absorptive absorbent core 25 disposed between the top sheet 23 and the back surface sheet 24.

The pair of wing portions 22 is formed by joining the top sheet 23 and the back surface sheet 24 at positions where the top sheet 23 and the back surface sheet 24 extend outward in a width direction from the absorbent main body 21.

The sheet member 6 has a rectangular shape. A length of the sheet member 6 is configured to be greater than a length of the absorbent main body 21, and a width of the sheet member 6 is configured to be greater than a width of the absorbent main body 21. The sheet member 6 is arranged to cover the sanitary napkin 2 on a back surface sheet 24 side, in a state in which the pair of wing portions are folded back toward a top sheet 23 side of the absorbent main body 21. In other words, the sanitary napkin 2 is arranged on an upper face of the sheet member 6 in a state in which the pair of wing portions 22 is folded back toward the top sheet 23.

As the sheet member 6, a film composed of a thermoplastic resin such as polyethylene, and a nonwoven fabric composed of a thermoplastic synthetic fiber such as polypropylene.

The sheet member 6 has predetermined coloring according to a type of the sanitary napkin 2 to be packaged.

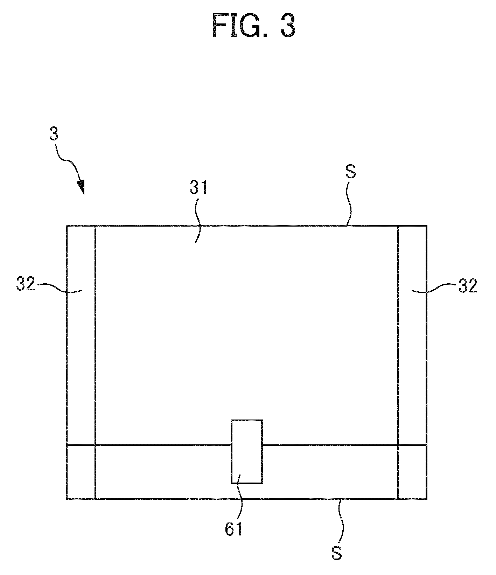

As shown in FIGS. 3 and 4, in the individual packaged good 3, the sheet member 6 and the sanitary napkin 2 are folded toward the top sheet 23 at a folding line S extending in the width direction of the absorbent main body 21, in a state in which the sanitary napkin 2 is arranged on the upper face of the sheet member 6.

In the present embodiment, two folding lines S are provided at positions substantially equally dividing the absorbent main body 21 into three in the longitudinal direction. In other words, the sanitary napkin 2 is packaged with the sheet member 6 in a triple-fold state.

The fixing tape 61 is attached on an outer face of the sheet member 6, on a first end side in the longitudinal direction thereof. The fixing tape 61 is attached in such a way that an end thereof protrudes from the first end side of the sheet member 6. A folded state of the individually packaged good 3 is maintained by attaching the end of the fixing tape 61 to the outer face of the sheet member 6 in a state in which the individually packaged good 3 is triple-folded with the first end side of the sheet member 6 directed outward.

As shown in FIG. 3, the individually packaged good 3 has a main body portion 31 in which the sanitary napkin in a folded state is covered with the sheet member 6, and a pair of extended portions 32 that are formed of the sheet member 6 extending from positions covering a pair of side portions of the sanitary napkin in the main body portion 31.

The main body portion 31 is configured such that the back surface sheet 24 side of the triple-folded sanitary napkin 2 is covered by the single-layered sheet member 6.

The pair of extended portions 32 is provided at positions corresponding to a pair of side portions extending in the longitudinal direction of the triple-folded sanitary napkin 2 (absorbent main body 21). The pair of extended portions 32 is configured such that the sheet member 6 extending from each of the pair of side portions of the absorbent main body 21 is layered into multiple layers (double or triple in the present embodiment) and the sheet member 6 thus layered is joined by heat sealing or pressure bonding.

From a viewpoint of stably housing the individually packaged goods 3 in the box body 5, when the individually packaged goods 3 is housed in the box body 3 as described later, an extension length of the pair of extended portions 32 is preferably 5 to 20 mm, and more preferably 10 to 15 mm, as described later.

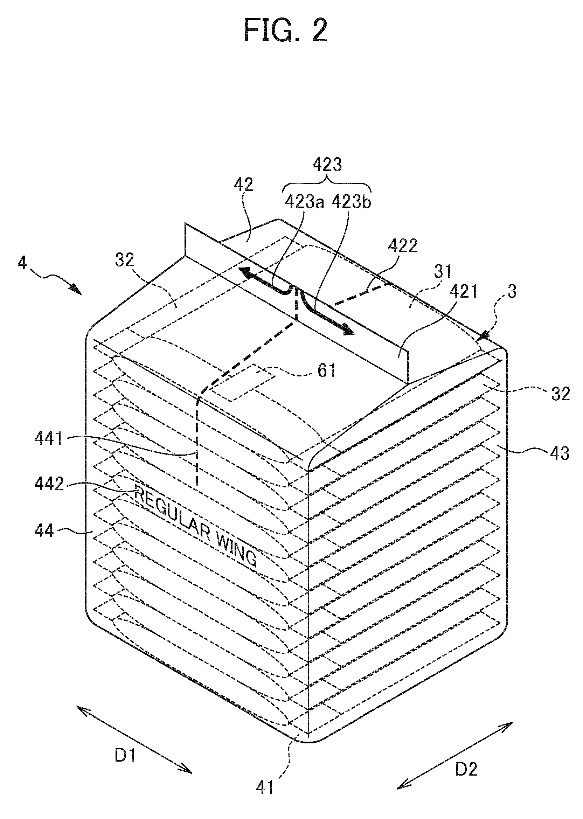

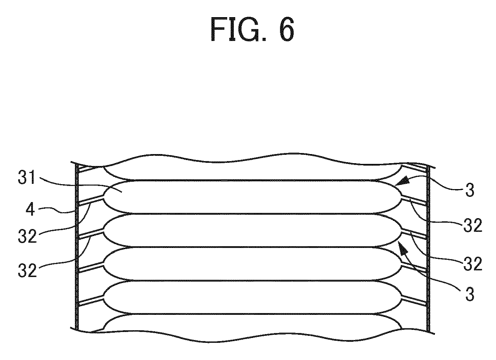

The bag body 4 houses the plurality of individually packaged goods 3 as shown in FIGS. 2 and 6. More specifically, the plurality of individually packaged goods 3 is housed in the bag body 4 such that a position of the pair of extended portions 32 is aligned in a thickness direction of the individually packaged goods 3.

In the present embodiment, the plurality of individually packaged goods 3 is housed in the bag body 4 as described below.

First, both ends of a belt-like sheet-shaped member are joined to form a tube-like shape with both ends of which are opened. Next, the plurality of individually packaged goods 3 is inserted into a tube-like part of the sheet-shaped member such that a stacking direction of the plurality of individually packaged goods 3 corresponds to a height direction of the tube-like sheet-shaped member. Thereafter, a first end side and a second end side of the tube-like sheet-shaped member are joined respectively. The bag body 4 housing the plurality of individually packaged goods 3 being stacked in the thickness direction is thus manufactured.

As shown in FIG. 2, in the bag body 4, the first end side and the second end side, in which the tube-like sheet-shaped member is joined, configure a bottom face 41 and a top face 42 respectively. In addition, portions of the sheet-shaped member directed at the pair of extended portions 32 of the plurality of individually packaged goods 3 thus stacked configure a pair of first side faces 43, 43; and portions of the sheet-shaped member directed at the folding line S of the plurality of individually packaged goods 3 configure a pair of second side faces 44, 44.

A length of an entire circumference of the tube-like sheet-shaped member in a peripheral direction is configured to be substantially the same as a length of periphery of the individually packaged good 3 in a state in which the pair of extended portions 32 is straightened. As a result, the plurality of individually packaged goods 3 is housed in the bag body 4 in a first state in which the pair of extended portions 32 is straightened outward from the main body portion 31 (see FIGS. 2 and 6). As used herein, the "state in which the pair of extended portions 32 is straightened outward" indicates a state in which an angle between an extension direction of the pair of extended portions 32 and a surface direction of the main body portion 31 is greater than 90 degrees. In other words, the first state includes, in addition to a state in which the pair of extended portions 32 extends straight along the surface direction of the main body portions 31, a state in which the pair of extended portions 32 extends at a predetermined angle with respect to the surface direction of the main body portions 31.

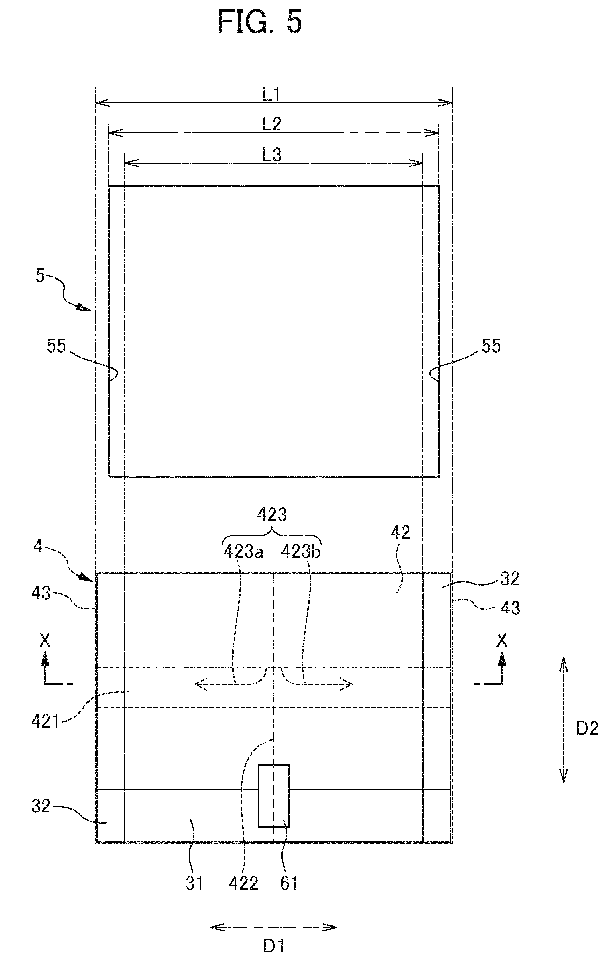

As shown in FIGS. 2 and 5, the upper face 42 of the bag body 4 includes: an upper sealed portion 421 where the tube-like sheet-shaped member is joined; a first perforated line 422; and a guide mark 423 provided on the upper sealed portion 421.

The upper sealed portion 421 is formed in a central part of the upper face 42 to extend in a direction D1 that is across (connects) the pair of extended portions 32 of the individually packaged good 3.

The first perforated line 422 is formed in a central part of the upper face 42 to extend in a direction D2 substantially orthogonal to the direction D1 that is across the pair of extended portions 32 of the individually packaged good 3.

For taking out the individually packaged good 3, the bag body 4 is opened from the first perforated line 422.

The guide mark 423 is composed of a first arrow mark 423a and a second arrow mark 423b provided in a central part of the upper sealed portion 421. A base side of the first arrow mark 423a is positioned at a central portion of the upper sealed portion 421 and a head side of the first arrow mark 423a is directed toward one side of the pair of first side faces 43. A base side of the second arrow mark 423b is positioned at the central portion of the upper sealed portion 421 and a head side of the second arrow mark 423b is directed toward the other side of the pair of first side faces 43.

The first arrow mark 423a and the second arrow mark 423b function as a guide mark for tearing the first perforated line 422 and the second perforated line 441 for opening the bag body 4.

As shown in FIG. 2, the second perforated line 441 is provided on each of the pair of second side faces 44. The pair of second perforated lines 441 is formed continuously from the first perforated line 422, and extends downward from an upper end of the second side face 44. In other words, a first one of the pair of second perforated lines 441 is connected to a first end of the first perforated line 422. A second one of the pair of second perforated lines 441 is connected to a second end of the first perforated line 422.

On a first one of the pair of second side faces 44, a product information 442 indicating a type and the like of the sanitary napkin 2 packaged in the individually packaged good 3 is provided.

The sheet-shaped member composing the bag body 4 is a translucent white synthetic resin film. This allows visual recognition of coloring of the sheet member 6 used in the individually packaged goods 3 housed in the bag body 4.

The box body 5 houses the bag body 4 housing the plurality of individually packaged goods 3 as shown in FIGS. 1, 7 and 8. The box body 5 is composed of a paper carton (cardboard box). More specifically, the box body 5 is configured by assembling a box by folding a flat cardboard at predetermined folding lines.

In addition, an outer face of the box body 5 has the same coloring as the sheet member 6 for the individually packaged goods 3 housed in the box body 5.

As shown in FIG. 1, the box body 5 includes: a box main body 51 with an open top; a pair of side flaps 52 and a flap 53 that cover the top; and an insertion portion 54 provided at a front end of the flap 53.

The box main body 51 has a substantially cubic shape. On an outer face of the box main body 51, a product information 511 indicating a type and the like of the sanitary napkin 2 housed in the box body 5 is provided.

The pair of side flaps 52 is provided on a pair of opposite peripheral edges among four peripheral edges constituting the opening of the top of the box main body 51. Each of the pair of side flaps 52 covers substantially half of the opening of the box main body 51.

The flap 53 is provided on one peripheral edge, on which the pair of side flaps 52 is not provided, of the four peripheral edges constituting the opening of the top of the box main body 51. The flap 53 covers the entire opening of the box main body 51.

The insertion portion 54 is formed by folding back the cardboard constituting the flap 53 at a front end of the flap 53.

As shown in FIGS. 1, 5, 8 and the like, the bag body 4 is housed in the box body 5 such that the pair of first side faces 43 is positioned to face vis-a-vis a pair of opposite internal faces 55 of the box body 5. In other words, in the present embodiment, the plurality of individually packaged goods 3 is housed in the box body 5 in a state of being stacked in the height direction of the box body 5.

Here, the product information 442 indicating a type and the like of the sanitary napkin 2 packaged in the individually packaged good 3 (see FIG. 2) is provided on the second side face 44; and the product information 511 indicating a type and the like of the sanitary napkin 2 to be housed in the box body 5 is provided on the outer face of the box body 5. In addition, the coloring provided on the sheet member 6 according to the type of the sanitary napkin 2 is the same as the coloring provided on the box body 5 housing the sanitary napkin 2, while the bag body 4 is composed of a translucent synthetic resin film allowing visual recognition of the coloring of the sheet member 6 from the outside.

Given this, in a case of manually housing the bag body 4, which houses the plurality of individually packaged goods 3, into the box body 5, a worker can correctly house the bag body 4 into the box body 5 by comparing the product information 442 provided on the second side face 44 of the bag body 4 with the product information 511 provided on the outer face of the box body 5.

The worker can house the bag body 4 into the box body 5 even more correctly by housing the bag body 4 into the box body 5 carrying the same coloring as the sheet member 6 that can be visually recognized through the bag body 4.

A relationship between the size of the bag body 4 and the size of the box body 5 is described hereinafter with reference to FIGS. 5 to 8. As described above, the bag body 4 houses the plurality of individually packaged goods 3 in the first state (see FIG. 6). As shown in FIG. 5, a length L1 between the pair of first side faces 43 (between edges of the pair of extended portions 32) of the bag body 4 in a state of housing the plurality of individually packaged goods 3 in the first state is configured to be greater than a length L2 between the pair of internal faces 55 of the box body 5. In addition, the length L2 between the pair of internal faces 55 of the box body 5 is configured to be greater than a length L3 of the main body portion 31 of the individually packaged good 3 in the direction across the pair of extended portions 32.

Given this, upon being housed into the box body 5, the bag body 4 is deformed due to the length L1 between edges of the pair of extended portions 32 that is greater than the length L2 between the internal faces of the box body. More specifically, the pair of extended portions 32 of the plurality of individually packaged goods 3 having been housed in the bag body 4 in the first state is made into the second state, being bent more inward than in the first state, due to pressure from the internal faces 55 of the box body 5. In a state in which the bag body 4 is housed in the box body 5, the internal faces 55 of the box body and the first side faces 43 of the bag body 4 are in contact with each other due to outward pressure from the pair of extended portions 32 of the plurality of individually packaged goods 3.

The box body 5 can package the bag body 4 by: housing the bag body 4 that houses the plurality of individually packaged goods 3 thereinside; covering the top of the box main body 51 with the pair of side flaps 52; further covering the top of the box main body 51 with the flap 53; and inserting the insertion portion 54 along the internal side face of the box main body 51.

Next, a mode of usage of the package 1 of the present embodiment is described.

In a case of using the package 1, the flap 53 and the pair of side flaps 52 are lifted to open the top of the box main body 51. Subsequently, the upper sealed portion 421 is pinched and pulled according to the guide mark 423 (the first arrow mark 423a and the second arrow mark 423b). The upper face 42 of the bag body 4 is thus opened along the first perforated line 422, and a part of the second side faces 44 is opened along the pair of second perforated lines 441. And then, fingers of a user are inserted through the opening formed on the bag body 4 to take out the individually packaged good 3 layered at the top of the stack of the plurality of individually packaged goods 3.

In a case of taking out the individually packaged good 3 from the box body 5, two fingers (for example, thumb and index finger) are inserted toward the pair of extended portions 32, respectively, to pinch the pair of extended portions 32 and take out the individually packaged good 3. Here, since the pair of extended portions 32 is smaller in thickness and greater in flexibility than the main body portion 31, the individually packaged good 3 can be easily pinched even through the internal faces 55 of the box body 5 and the pair of extended portions 32 are in contact with each other across the bag body 4.

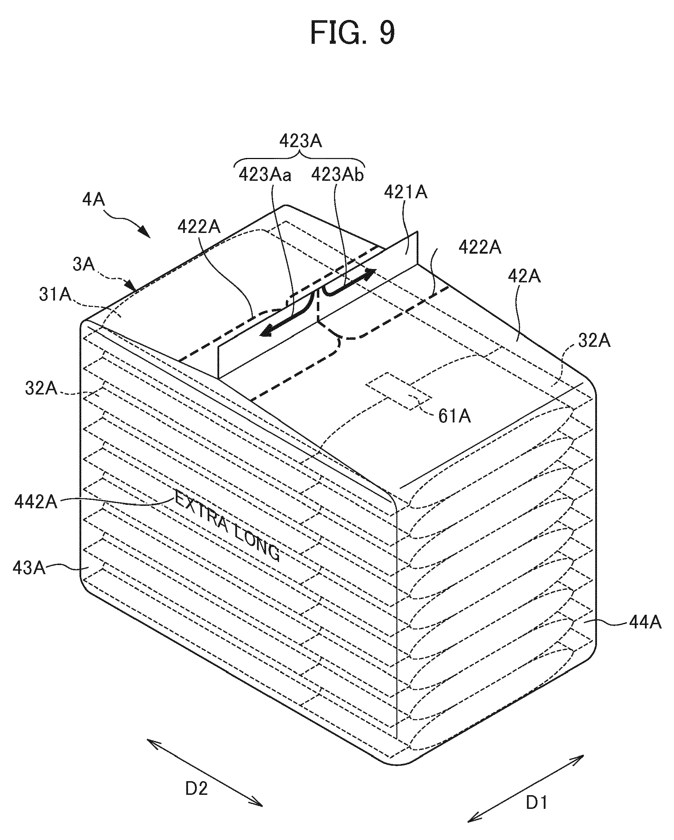

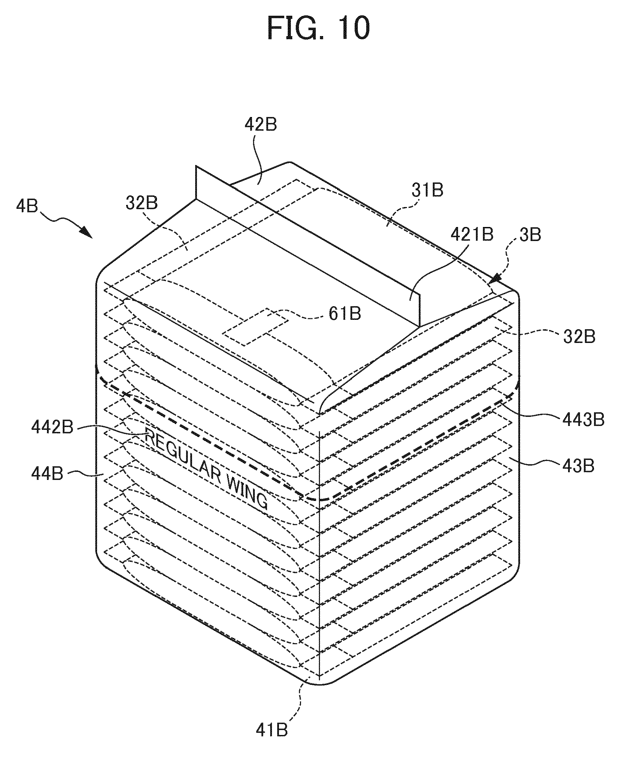

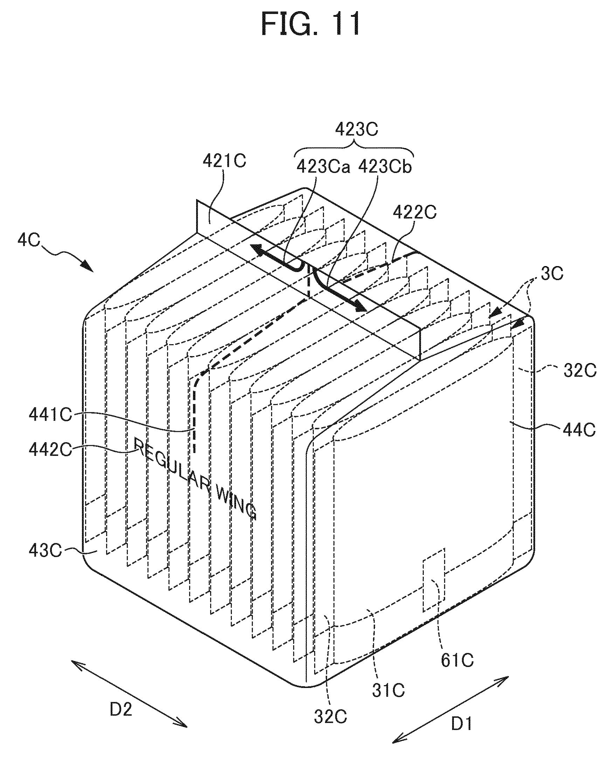

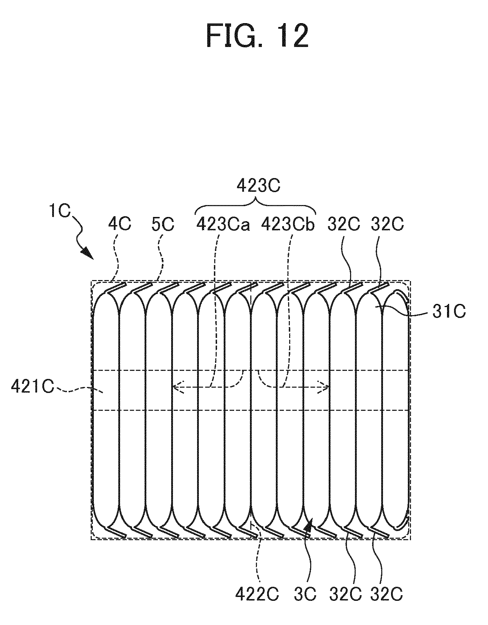

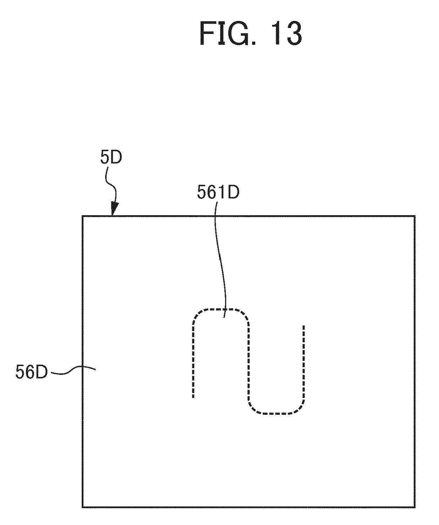

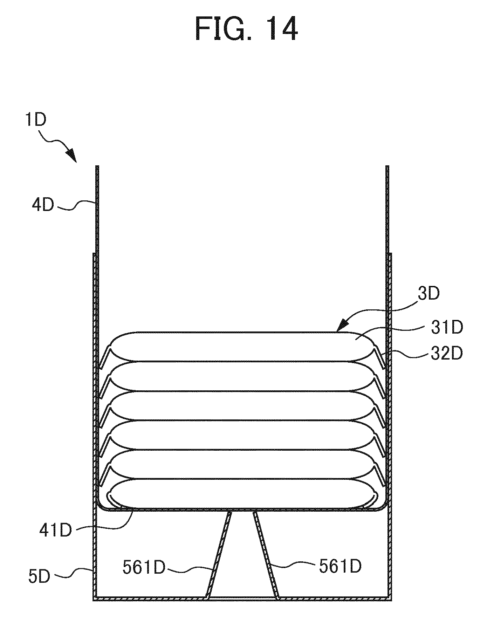

The above-described package 1 according to the first embodiment produces the following effects.
(1) The length L1 between ends of the pair of extended portions of the individually packaged good 3, in a state in which the pair of extended portions 32 is straightened, is configured to be greater than the length L2 between the pair of internal faces 55 of the box body 5. This makes it possible to support the plurality of individually packaged goods 3 by the pair of internal faces 55 of the box body 5. As a result, the individually packaged goods 3 can be preferably supported by the pair of internal faces 55 inside the box body 5 and movement of the individually packaged goods 3 inside the box body 5 can be prevented even if there is only a few individually packaged goods 3 left in the box body 5. The individually packaged goods 3 housed in the box body 5 can thus be easily taken out.
   In addition, in a case of taking out the individually packaged good 3 from the box body 5, two fingers (for example, thumb and index finger) are inserted toward the pair of extended portions 32, respectively, to pinch the pair of extended portions 32 and take out the individually packaged good 3. Here, since the pair of extended portions 32 is smaller in thickness and greater in flexibility than the main body portion 31, the individually packaged good 3 can be easily pinched even through the internal faces 55 of the box body 5 and the pair of extended portions 32 are in contact with each other across the bag body 4.
(2) In the individually packaged good 3 of the sanitary napkin 2, the sanitary napkin 2 folded at the folding lines S is packaged with the sheet member 6. Therefore, a stiffness of the individually packaged good 3 is greater in a case of applying a force from the folding line S side than in a case of applying a force from a side along the longitudinal direction of the sanitary napkin 2. In other words, the individually packaged good 3 is more easily deformable in a case of applying a force from the side along the longitudinal direction of the sanitary napkin 2 than in a case of applying a force from the folding line S side. Given this, the pair of extended portions 32 is provided on the sides along the longitudinal direction of the sanitary napkin 2. Since the individually packaged good 3 is easily deformed when fingers are inserted toward the pair of extended portions 32, the individually packaged good 3 can be taken out more easily.
(3) The pair of extended portions 32 is configured by joining the multi-layered sheet member 6. This can provide a preferable stiffness to the pair of extended portions 32. As a result, the individually packaged goods 3 can be preferably supported by the box body 5, and movement of the individually packaged goods 3 inside the box body 5 can further be prevented.
(4) The bag body 4 is housed in the box body 5 in a state in which the plurality of individually packaged goods 3 is stacked in the height direction of the box body 5. This facilitates insertion of fingers toward the pair of extended portions 32 for taking out the individually packaged good 3 from the upper face 42 side of the bag body 4. Convenience in taking out of the individually packaged goods 3 can thus further be improved.
(5) The plurality of individually packaged goods 3 is housed in the bag body 4 in the first state in which the pair of extended portions 32 is straightened outward; and housed in the box body 5 in the second state in which the pair of extended portions 32 is bent more inward than in the first state. As a result, in a state in which the bag body 4 is housed in the box body 5, the internal faces 55 of the box body and the first side faces 43 of the bag body 4 are in contact with each other due to outward pressure from the pair of extended portions 32 of the plurality of individually packaged goods 3. As a result, the bag body 4 housed in the box body 5 does not easily move inside the box body 5. In addition, the plurality of individually packaged goods 3 housed in the bag body 4 can thus be supported by the pair of internal faces 55 of the box body 5 through the bag body 4. As a result, the individually packaged goods 3 can be preferably supported by the pair of internal faces 55 of the box body 5 and movement of the individually packaged goods 3 inside the box body 5 can be prevented even if there is only a few individually packaged goods 3 left in the bag body 4. The individually packaged goods 3 housed in the box body 5 can thus be easily taken out.
(6) The first perforated line 422 is provided on the upper face 42 of the bag body 4. As a result, the plurality of individually packaged goods 3 housed in the bag body 4 can thus be taken out without taking out the bag body 4 from the box body 5.
(7) The pair of second perforated lines that is connected to the first perforated line 422 is provided on the pair of second side faces 44 of the bag body 4. As a result, the upper face and a part of the pair of second side faces 44 of the bag body 4 can be opened, thereby further facilitating taking out of the individually packaged goods 3 housed in the bag body 4.
(8) The first perforated line 422 is formed to extend in the direction D2 substantially orthogonal to the direction D1 that is across the pair of extended portions 32. As a result, the upper face 42 of the bag body 4 can be opened for a substantially entire length in a direction across the folding lines S, S of the high-rigidity main body portion 31 (longitudinal direction), and therefore the individually packaged good 3 can be easily taken out by pinching the pair of extended portions 32.
(9) The box body 5 is configured to include the box main body 51 with an open top, and the flap 53 that covers the open top. Given this, even after opening the upper face 42 of the bag body 4 housed in the box body 5 and taking out a part of the individually packaged goods 3, the upper face 42 of the bag body 4 can be covered with the flap portion 53 and hygiene of the individually packaged goods 3 housed in the box body 5 (bag body 4) can be maintained.
   Next, a second embodiment of the present invention is described hereinafter with reference to FIG. 9. FIG. 9 is a perspective view illustrating a bag body 4A according to the second embodiment. In the following description of the second embodiment, the same components are denoted by the same reference numerals, and description thereof will be omitted or simplified.
   The package of the second embodiment is different from the first embodiment mainly in a shape of the individually packaged good 3 and a configuration of the first perforated line 422A.
   The individually packaged body 3A of the second embodiment is configured such that a triple-folded sanitary napkin greater in size than the sanitary napkin of the first embodiment is packaged with a sheet member, and has a rectangular shape in planar view. In the individually packaged body 3A, the pair of extended portions 32A extends from sides along the longitudinal direction of the individually packaged goods 3A.
   In addition, in the second embodiment, the upper sealed portion 421A is formed to extend in a direction D1 that is across the pair of extended portions 32A. Two first perforated lines 422A are provided to extend along an extension direction of the upper sealed portion 421A (D1) and join together at a central portion of the upper face 42A, in a manner to sandwich the upper sealed portion 421A. The product information 442A is provided on the first side face 43A.
   The second embodiment produces the following effects, in addition to the abovementioned effects (1) to (9).
(10) The first perforated lines 422A are provided to sandwich the upper sealed portion 421A. By pinching and pulling the upper sealed portion 421A along the first perforated lines 422A according to the guide mark 423, a wider opening than in the first embodiment can be formed. The individually packaged goods 3A housed in the bag body 4A can thus be taken out more easily.
(11) The first perforated line 422A is formed to extend in the direction D1 that is across the pair of extended portions 32A. As a result, the upper face 42A of the bag body 4A can be easily opened up to a position corresponding to the pair of extended portions 32A, thereby facilitating pinching of the pair of extended portions 32A.
   Next, a third embodiment of the present invention is described hereinafter with reference to FIG. 10. FIG. 10 is a perspective view illustrating a bag body 4B according to the third embodiment.
   The package of the third embodiment is different from the first embodiment in providing a third perforated line 443B in place of the first perforated line 422 and the second perforated line 441.
   The third perforated line 443B is formed on the pair of first side faces 43B and the pair of second side faces 44B of the bag body 4B. The third perforated line 443B extends in a direction substantially orthogonal to the height direction of the bag body 4B. In other words, the third perforated line 443B is formed continuously for an entire periphery of the bag body 4B.
   The third embodiment produces the following effects, in addition to the abovementioned effects (1) to (5) and (9).
(12) The third perforated line 443B extending in a direction substantially orthogonal to the height direction of the bag body 4B is provided on the first side faces 43B and the second side faces 44B. This allows split-off of the upper face 42B of the bag body 4B from the third perforated line 443B. By thus opening up the upper face 42B of the bag body 4B, the individually packaged goods 3 can be taken out more easily.
   Next, a fourth embodiment of the present invention is described hereinafter with reference to FIGS. 11 and 12. FIG. 11 is a perspective view illustrating a bag body 4C according to the fourth embodiment. FIG. 12 is a plan view illustrating a state in which the bag body 4C is housed in the box body 5C.
   The package 1C of the fourth embodiment is different from the first embodiment mainly in arrangement of the individually packaged goods 3C housed inside the bag body 4C and arrangement of the first perforated line 422C.
   In the fourth embodiment, as shown in FIGS. 11 and 12, the plurality of individually packaged goods 3C is housed in the bag body 4C such that the longitudinal direction of the sanitary napkins packaged in the individually packaged goods 3C corresponds to the height direction of the box body 5C.
   The first perforated line 422C extends in the direction D1 that is across the pair of extended portions 32C.
   The fourth embodiment produces the following effects, in addition to the abovementioned effects (1) to (3), (5) to (7), (9), and (11).
   Next, a fifth embodiment of the present invention is described hereinafter with reference to FIGS. 13 and 14. FIG. 13 is a bottom view of the box body 5D according to the fifth embodiment. FIG. 14 is a diagram illustrating a used state of the package 1D of the fifth embodiment.
   The package 1D of the fifth embodiment is different from the first embodiment in a configuration of the box body 5C.
   In the fifth embodiment, as shown in FIGS. 13 and 14, a push-up portion 561D is provided on a bottom face 56D of the box body 5D. The push-up portion 561D is formed by providing an S-shaped perforated line on the cardboard constituting the bottom face 56D of the box body 5D.
   In the fifth embodiment, as shown in FIG. 15, when there is only a few individually packaged goods 3D in the box body 5D, the push-up portion 561D can be pressed up into the box body 5D by tearing from the perforated line provided on the bottom face 56D of the box body 5D, to thereby push up the bag body 4D.
   The fifth embodiment produces the following effects, in addition to the abovementioned effects (1) to (9).
(13) The push-up portion 561D is provided on the bottom face 56D of the box body 5D. Given this, when there is only a few individually packaged goods 3D in the box body 5D, by pushing up the bottom face of the bag body 4D by means of the push-up portion 561D, the individually packaged goods 3D can be easily taken out from the top of the box body 5D until the last one.

The preferred embodiments of the package according to the present invention have been described above; however, the present invention is not limited thereto and can be embodied in various modes.

For example, in the first embodiment, the length L1 between ends of the pair of extended portions 32 of the individually packaged good 3, in a state of being housed in the bag body 4, is configured to be greater than the length L2 between the pair of internal faces 55 of the box body 5; however, the present invention is not limited thereto. In other words, the length between ends of the pair of extended portions in a state of being housed in the bag body can also be configured to be as if substantially the same, as long as slightly longer than the length between the pair of internal faces of the box body 5.

In addition, in the first to fifth embodiments, the present invention is employed in sanitary napkins; however, the present invention is not limited thereto. The present invention can also be employed in interlabial pads or incontinence pads, as the absorbent articles.

Furthermore, in the first to fifth embodiments, a translucent white synthetic resin film is used for the bag body; however, the present invention is not limited thereto. For example, a synthetic resin film of the same coloring as the sheet member for the individually packaged goods can also be used for the bag body. Housing the bag body into the box body carrying the same coloring allows correct selection in housing of the bag body into the box body. By making the design of the bag body and the box body consistent, the package of the present invention can provide a user with an impression of cleanliness and smartness like fancy household goods.

In addition, in the first to fifth embodiments, a single bag body 4 is housed in a single box body 5; however, the present invention is not limited thereto. In other words, a plurality of bag bodies can be housed in a single box body. In this case, the plurality of bag bodies can house either the same type of individually packaged goods, or different products (for example, a bag body housing the individually packaged goods of sanitary napkin and a bag body housing moist wipes). It is required that at least one of the plurality of bag bodies has the configuration of the present invention.

Moreover, a sheet material printed with a predetermined text such as instructions for use and new product information can be inserted between the box body 5 and the bag body 4. In this case, the sheet material with the text does not get into direct contact with the individually packaged goods, thereby allowing information provision to a user while maintaining hygiene of the individually packaged goods.

Product information and the like can also be provided in Braille on the outer face of the box body and/or the upper face of the bag body. Information regarding the individually packaged goods can thus be properly provided to visually challenged users.

## Claims

1. A package (1) for individually packaged good (3) of an absorbent article (2), comprising:
an individually packaged good (3) including the absorbent article (2) packaged with a sheet member (6), a main body portion (31) in which the absorbent article (2) is covered with the sheet member (6), and a pair of extended portions (32) that are formed of the sheet member (6) extending from positions covering a pair of side portions of the absorbent article (2) in the main body portion (31);
**characterized by** a bag body (4) housing a plurality of the individually packaged goods (3); and
a box body (5) that houses the bag body (4) housing the plurality of individually packaged goods (3), of which an upper face can be opened,
wherein the plurality of individually packaged goods (3) is housed in the bag body (4) such that a position of the pair of extended portions (32) is aligned in a thickness direction of the individually packaged goods (3);
wherein the plurality of individually packaged goods (3) is housed in the bag body (4) in a first state in which the pair of extended portions is straightened outward from the main body portion (31);
the bag body (4) housing the plurality of individually packaged goods (3) is housed in the box body (5) such that a pair of side faces (43), to which the pair of extended portions is directed, is positioned to face a pair of opposite internal faces (55) of the box body;
a length between ends of the pair of extended portions (32) of the individually packaged good (3), in a state in which the pair of extended portions (32) is straightened, is greater than a distance between the pair of internal faces (55) of the box body; and
the bag body (4) housing the plurality of individually packaged goods (3) is housed in the box body (5) in a second state in which the pair of extended portions (32) is bent more inward than in the first state.

2. The package (1) for individually packaged good (3) of the absorbent article (2) according to claim 1,
wherein the absorbent article (2) has an elongated absorbent main body (21);
the individually packaged good (3) is configured to be packaged with the sheet member (3) in a state in which the absorbent article (21) is folded in a longitudinal direction at a fold line (S) along a width direction of the absorbent main body (21); and
the pair of extended portions (32) is provided at the pair of side portions extending in the longitudinal direction of the absorbent article (2) and configured such that portions of the sheet member (6) extending from the pair of side portions are joined.

3. The package (1) for individually packaged good (3) of the absorbent article (2) according to claim 1 or 2,
wherein the bag body (4) is housed in the box body (5) in a state in which the plurality of individually packaged goods (3) is stacked in a height direction of the box body (5).

4. The package (1) for individually packaged good (3) of the absorbent article (2) according to any one of claims 1 to 3,
wherein the bag body (4) has a first perforated line (422) formed on an upper face thereof in a state of being housed in the box body (5).

5. The package (1) for individually packaged good (3) of the absorbent article (2) according to claim 4,
wherein the bag body (4) further has a second perforated line (441) formed on a side face (44) thereof, the second perforated line (441) being connected to the first perforated line (422).

6. The package (1) for individually packaged good (3) of the absorbent article (2) according to claim 4 or 5,
wherein the first perforated line (422) extends in a direction substantially orthogonal to a direction across the pair of extended portions (32).

7. The package (1) for individually packaged good (3) of the absorbent article (2) according to claim 4 or 5,
wherein the first perforated line (422A) extends in a direction across the pair of extended portions (32A).

8. The package (1) for individually packaged good (3) of the absorbent article (2) according to any one of claims 1 to 7,
wherein the bag body (4B) further has a third perforated line (443B) that is formed on a side face (44B) of the bag body (4B), the third perforated line (443B) extending in a direction substantially orthogonal to a height direction of the bag body (4).

9. The package (1) for individually packaged good (3) of the absorbent article (2) according to any one of claims 1 to 8,
wherein the bag body (4) is formed of a white sheet-like member.

10. The package (1) for individually packaged good (3) of the absorbent article (2) according to any one of claims 1 to 9,
wherein the box body (4) includes a push-up portion (561D) formed on a bottom face (56D) thereof, the push-up portion (561D) allowing push-up of the bag body (4D).

## Patentansprüche

1. Verpackung (1) für einzeln verpackte Waren (3) eines absorbierenden Artikels (2), die Folgendes umfasst:
eine einzeln verpackte Ware (3), die Folgendes einschließt: den absorbierenden Artikel (2), der mit einem Lagenelement (6) verpackt ist, einen Hauptkörperabschnitt (31), in dem der absorbierende Artikel (2) mit dem Lagenelement (6) bedeckt ist, und ein Paar an verlängerten Abschnitten (32), die aus dem Lagenelement (6) gebildet sind, das sich von Positionen erstreckt, die ein Paar an Seitenabschnitten des absorbierenden Artikels (2) in dem Hauptkörperabschnitt (31) bedecken;
**gekennzeichnet durch** einen Taschenkörper (4), der eine Vielzahl der einzeln verpackten Waren (3) aufnimmt; und
einen Gehäusekörper (5), der den Taschenkörper (4) aufnimmt, der die Vielzahl der einzeln verpackten Waren (3) aufnimmt, von dem eine obere Fläche geöffnet werden kann,
wobei die Vielzahl an einzeln verpackten Waren (3) in dem Taschenkörper (4) aufgenommen ist, sodass eine Position des Paars an verlängerten Abschnitten (32) in einer Dickenrichtung der einzeln verpackten Waren (3) ausgerichtet ist;
wobei die Vielzahl an einzeln verpackten Waren (3) in dem Taschenkörper (4) in einem ersten Zustand aufgenommen ist, in dem das Paar an verlängerten Abschnitten von dem Hauptkörperabschnitt (31) nach außen gestreckt ist;
der Taschenkörper (4), der die Vielzahl an einzeln verpackten Waren (3) aufnimmt, in dem Gehäusekörper (5) aufgenommen ist, sodass ein Paar an Seitenflächen (43), zu denen das Paar an verlängerten Abschnitten ausgerichtet ist, so positioniert ist, dass es einem Paar an gegenüberliegenden inneren Flächen (55) des Gehäusekörpers zugewandt ist;
eine Länge zwischen Enden des Paars an verlängerten Abschnitten (32) der einzeln verpackten Ware (3), in einem Zustand, in dem das Paar an verlängerten Abschnitten (32) gestreckt ist, größer ist als ein Abstand zwischen dem Paar an inneren Flächen (55) des Gehäusekörpers; und
der Taschenkörper (4), der die Vielzahl an einzeln verpackten Waren (3) aufnimmt, in dem Gehäusekörper (5) in einem zweiten Zustand aufgenommen ist, in dem das Paar an verlängerten Abschnitten (32) weiter nach innen gebogen ist als in dem ersten Zustand.

2. Verpackung (1) für einzeln verpackte Waren (3) des absorbierenden Artikels (2) nach Anspruch 1,
wobei der absorbierende Artikel (2) einen länglichen absorbierenden Hauptkörper (21) aufweist;
die einzeln verpackte Ware (3) konfiguriert ist, um mit dem Lagenelement (3) in einem Zustand verpackt zu werden, in dem der absorbierende Artikel (21) in einer Längsrichtung an einer Faltlinie (S) entlang einer Breitenrichtung des absorbierenden Hauptkörpers (21) gefaltet ist; und
das Paar an verlängerten Abschnitten (32) an dem Paar an Seitenabschnitten bereitgestellt ist, die sich in der Längsrichtung des absorbierenden Artikels (2) erstrecken, und derart konfiguriert ist, dass Abschnitte des Lagenelements (6), das sich von dem Paar an Seitenabschnitten erstreckt, verbunden sind.

3. Verpackung (1) für einzeln verpackte Waren (3) des absorbierenden Artikels (2) nach Anspruch 1 oder 2,
wobei der Taschenkörper (4) in dem Gehäusekörper (5) in einem Zustand aufgenommen ist, in dem die Vielzahl an einzeln verpackten Waren (3) in einer Höhenrichtung des Gehäusekörpers (5) gestapelt ist.

4. Verpackung (1) für einzeln verpackte Waren (3) des absorbierenden Artikels (2) nach einem der Ansprüche 1 bis 3,
wobei der Taschenkörper (4) eine erste perforierte Linie (422), die auf einer oberen Seite davon ausgebildet ist, in einem Zustand zur Aufnahme in dem Gehäusekörper (5) aufweist.

5. Verpackung (1) für einzeln verpackte Waren (3) des absorbierenden Artikels (2) nach Anspruch 4,
wobei der Taschenkörper (4) weiter eine zweite perforierte Linie (441) aufweist, die auf einer Seitenfläche (44) davon ausgebildet ist, wobei die zweite perforierte Linie (441) mit der ersten perforierten Linie (422) verbunden ist.

6. Verpackung (1) für einzeln verpackte Waren (3) des absorbierenden Artikels (2) nach Anspruch 4 oder 5,
wobei sich die erste perforierte Linie (422) in einer Richtung erstreckt, die im Wesentlichen senkrecht zu einer Richtung über das Paar an verlängerten Abschnitten (32) vorliegt.

7. Verpackung (1) für einzeln verpackte Waren (3) des absorbierenden Artikels (2) nach Anspruch 4 oder 5,
wobei sich die erste perforierte Linie (422A) in einer Richtung über das Paar an verlängerten Abschnitten (32A) erstreckt.

8. Verpackung (1) für einzeln verpackte Waren (3) des absorbierenden Artikels (2) nach einem der Ansprüche 1 bis 7,
wobei der Taschenkörper (4B) weiter eine dritte perforierte Linie (443B) aufweist, die an einer Seitenfläche (44B) des Taschenkörpers (4B) ausgebildet ist, wobei sich die dritte perforierte Linie (443B) in einer Richtung erstreckt, die im Wesentlichen senkrecht zu einer Höhenrichtung des Taschenkörpers (4) vorliegt.

9. Verpackung (1) für einzeln verpackte Waren (3) des absorbierenden Artikels (2) nach einem der Ansprüche 1 bis 8,
wobei der Taschenkörper (4) aus einem weißen lagenartigen Element ausgebildet ist.

10. Verpackung (1) für einzeln verpackte Waren (3) des absorbierenden Artikels (2) nach einem der Ansprüche 1 bis 9,
wobei der Gehäusekörper (4) einen Hochdrückabschnitt (561D) einschließt, der an einer unteren Fläche (56D) davon ausgebildet ist, wobei der Hochdrückabschnitt (561D) das Hochdrücken des Taschenkörpers (4D) erlaubt.

## Revendications

1. Emballage (1) pour un produit emballé individuellement (3) d'un article absorbant (2), comportant :
un produit emballé individuellement (3) comprenant l'article absorbant (2) emballé avec un élément de type feuille (6), une partie formant corps principal (31) dans laquelle l'article absorbant (2) est recouvert au moyen de l'élément de type feuille (6), et une paire de parties allongées (32) qui sont formées à partir de l'élément de type feuille (6) s'étendant depuis des positions recouvrant une paire de parties latérales de l'article absorbant (2) dans la partie formant corps principal (31) ;
**caractérisé par** un corps formant pochette (4) recevant une pluralité des produits emballés individuellement (3) ; et
un corps formant boîte (5) qui reçoit le corps formant pochette (4) recevant la pluralité de produits emballés individuellement (3), dont une face supérieure peut être ouverte,
dans lequel la pluralité de produits emballés individuellement (3) est reçue dans le corps formant pochette (4) de telle sorte qu'une position de la paire de parties allongées (32) est alignée dans une direction allant dans le sens de l'épaisseur des produits emballés individuellement (3) ;
dans lequel la pluralité de produits emballés individuellement (3) est reçue dans le corps formant pochette (4) dans un premier état dans lequel la paire de parties allongées est redressée vers l'extérieur depuis la partie formant corps principal (31) ;
le corps formant pochette (4) recevant la pluralité de produits emballés individuellement (3) est reçu dans le corps formant boîte (5) de telle sorte qu'une paire de faces latérales (43), vers laquelle la paire de parties allongées est dirigée, est positionnée à des fins d'orientation vers une paire de faces internes opposées (55) du corps formant boîte ;
une longueur entre des extrémités de la paire de parties allongées (32) du produit emballé individuellement (3), dans un état dans lequel la paire de parties allongées (32) est redressée, est supérieure par rapport à une distance entre la paire de faces internes (55) du corps formant boîte ; et
le corps formant pochette (4) recevant la pluralité de produits emballés individuellement (3) est reçue dans le corps formant boîte (5) dans un deuxième état dans lequel la paire de parties allongées (32) est courbée plus vers l'intérieur que dans le premier état.

2. Emballage (1) pour un produit emballé individuellement (3) de l'article absorbant (2) selon la revendication 1,
dans lequel l'article absorbant (2) a un corps principal absorbant allongé (21) ;
le produit emballé individuellement (3) est configuré pour être emballé avec l'élément de type feuille (3) dans un état dans lequel l'article absorbant (21) est plié dans une direction allant dans le sens longitudinal au niveau d'une ligne de pliage (S) le long d'une direction allant dans le sens de la largeur du corps principal absorbant (21) ; et
la paire de parties allongées (32) est mise en oeuvre au niveau de la paire de parties latérales s'étendant dans la direction allant dans le sens longitudinal de l'article absorbant (2) et configurée de telle sorte que des parties de l'élément de type feuille (6) s'étendant depuis la paire de parties latérales sont jointes.

3. Emballage (1) pour un produit emballé individuellement (3) de l'article absorbant (2) selon la revendication 1 ou la revendication 2,
dans lequel le corps formant pochette (4) est reçu dans le corps formant boîte (5) dans un état dans lequel la pluralité de produits emballés individuellement (3) est empilée dans une direction allant dans le sens de la hauteur du corps formant boîte (5).

4. Emballage (1) pour un produit emballé individuellement (3) de l'article absorbant (2) selon l'une quelconque des revendications 1 à 3,
dans lequel le corps formant pochette (4) a une première ligne perforée (422) formée sur une face supérieure de celui-ci dans un état dans lequel il est reçu dans le corps formant boîte (5).

5. Emballage (1) pour un produit emballé individuellement (3) de l'article absorbant (2) selon la revendication 4,
dans lequel le corps formant pochette (4) a par ailleurs une deuxième ligne perforée (441) formée sur une face latérale (44) de celui-ci, la deuxième ligne perforée (441) étant raccordée à la première ligne perforée (422).

6. Emballage (1) pour un produit emballé individuellement (3) de l'article absorbant (2) selon la revendication 4 ou la revendication 5,
dans lequel la première ligne perforée (422) s'étend dans une direction sensiblement perpendiculaire par rapport à une direction allant en travers de la paire de parties allongées (32).

7. Emballage (1) pour un produit emballé individuellement (3) de l'article absorbant (2) selon la revendication 4 ou la revendication 5,
dans lequel la première ligne perforée (422A) s'étend dans une direction allant en travers de la paire de parties allongées (32A).

8. Emballage (1) pour un produit emballé individuellement (3) de l'article absorbant (2) selon l'une quelconque des revendications 1 à 7,
dans lequel le corps formant pochette (4B) a par ailleurs une troisième ligne perforée (443B) qui est formée sur une face latérale (44B) du corps formant pochette (4B), la troisième ligne perforée (443B) s'étendant dans une direction sensiblement perpendiculaire par rapport à une direction allant dans le sens de la hauteur du corps formant pochette (4).

9. Emballage (1) pour un produit emballé individuellement (3) de l'article absorbant (2) selon l'une quelconque des revendications 1 à 8,
dans lequel le corps formant pochette (4) est formé à partir d'un élément similaire à une feuille blanche.

10. Emballage (1) pour un produit emballé individuellement (3) de l'article absorbant (2) selon l'une quelconque des revendications 1 à 9,
dans lequel le corps formant pochette (4) comprend une partie de poussée (561D) formée sur une face inférieure (56D) de celui-ci, la partie de poussée (561D) permettant de pousser le corps formant pochette (4D).
